# EUROPÄISCHE PATENTSCHRIFT

(11) **EP 0 764 658 B1**
(45) Veröffentlichungstag und Bekanntmachung des Hinweises auf die Patenterteilung: **02.01.2002**
(21) Anmeldenummer: 95810596.7
(22) Anmeldetag: 22.09.1995
(51) Int. Cl.: C07K 16/06, C07K 16/08, C07K 16/12, C07K 16/18, A61K 39/40, A61K 39/42

(54) **Verfahren zur Gewinnung von Immunglobulinen aus Fraktionen, die bei der Fraktionierung von menschlichem Blutplasma entstehen**
Process for obtaining immunoglobulins from fractions originating from the fractionation of human blood plasma
Procédé d'obtention d'immunoglobulines à partir de fractions provenant du fractionnement de plasma sanguin humain

(43) Veröffentlichungstag der Anmeldung: 26.03.1997
(73) Patentinhaber: ZLB Bioplasma AG, 3014 Bern (CH)
(72) Erfinder: Amstutz, Hanspeter, CH-3065 Bolligen (CH); Lerch, Peter G., CH-3007 Bern (CH); Morgenthaler, Jean-Jacques, CH-3067 Boll (CH)
(74) Vertreter: BOVARD AG - Patentanwälte

(56) Entgegenhaltungen:
- EP-A- 0 201 004
- EP-A- 0 345 543
- US-A- 4 232 004

## Beschreibung

Die vorliegende Erfindung betrifft ein Verfahren zur Gewinnung von Immunglobulinen aus Fraktionen, die bei den heute üblichen Plasmafraktionierverfahren zur Herstellung von klinischen Präparaten nicht verwertet werden, oder zumindest nicht bei der Herstellung von Immunglobulinpräparaten zum Einsatz gelangen.

Im menschlichen Blutplasma findet man über hundert verschiedene Proteine. Einige von ihnen können aus Pools von Spenderplasma durch Fraktionierung gereinigt und als therapeutische Produkte verwendet werden. Folgende Beispiele sind bekannt: Albumin wird zum Ausgleich eines onkotischen Defizits bei Hypoproteinämie oder bei Hypovolämie eingesetzt. Blutgerinnungsfaktor VIII bzw. IX wird zur Prophylaxe und Therapie von Blutungen bei Haemophilie A bzw. B verabreicht. Immunglobuline werden bei Antikörpermangelkrankheiten zur Prophylaxe und Therapie von Infektionen sowie bei idiopathischer thrombozytopänischer Purpura angewendet. Immunglobuline aus ausgewählten Spendern mit hohen Titern an spezifischen Immunglobulinen werden als Hyperimmunoglobulin-Präparate zur Prophylaxe und Behandlung von spezifischen Infektionen wie z.B. Hepatitis A oder B eingesetzt.

Die Isolierung von therapeutisch verwendbaren Plasmaproteinen kann zum Beispiel nach den bekannten Methoden der Ethanol-Fraktionierung (Cohn, E.G., et al., J. Am. Chem. Soc., 68, 459, 1946 sowie Kistler, P. und Nitschmann, H., Vox Sang., 7, 414, 1962) geschehen. Mit beiden Verfahren können grosse Mengen von funktionellen Plasmaproteinen wie Albumin oder Immunglobuline isoliert werden, die in geeigneten Formulierungen klinisch nutzbringend einsetzbar sind. Es entstehen aber bei der Aufarbeitung nach diesen Methoden Niederschläge bzw. Überstände, die bei der konventionellen Verarbeitung nicht verwendet werden. Die Zusammensetzung dieser Fraktionen ist sehr unterschiedlich.

Während beispielsweise humanes Apolipoprotein A-I (Apo A-I) bei der Ausfällung von Blutplasma mit 19% Ethanol bei einem pH-Wert von 5.8 zu etwa gleichen Teilen im Überstand a (etwa 50% des Plasma-ApoA-I) und im Niederschlag A (etwa 40%) zu finden ist, findet sich das gleiche Protein nach den nächsten Fraktionierungsschritten nach Kistler und Nitschmann dann in denjenigen Fraktionen, die bisher nicht kommerziell genutzt wurden: Niederschlag IV und Niederschlag B (je etwa 40%) (Lerch et al., Protides of the Biological Fluids, 36, 409, 1989).

Ein ähnliches Verteilungsmuster ergibt sich für das Kupfer-Transportprotein Caeruloplasmin. Man findet nach der Fraktionierung 20% des Ausgangsmaterials in Niederschlag IV und 40% in Niederschlag B.

Transferrin ist ein Beispiel für ein Plasmaprotein, das sich bei den gleichen Fraktionierungsverfahren zu praktisch 100% in Niederschlag IV anreichert, während Albumin zu 80% im heute genutzten Niederschlag C zu finden ist.

Immunglobuline können mit der Kistler-Nitschmann- oder der Cohn-Fraktionierung zu 50 - 60% im Niederschlag GG gewonnen werden. Die verbleibenden 30 - 40% verteilen sich nach diesen Verfahren auf Niederschlag IV (5%), Niederschlag B (30%) und Filtrat GG (5%).

Die oben genannten Prozentzahlen sollen die Grössenordnung der Verteilung illustrieren und sind nicht als Einschränkung zu verstehen; sie sind je nach den verwendeten Bedingungen und Methoden variabel.

Diese Beispiele zeigen, dass zum Teil beträchtliche Mengen an therapeutisch nutzbaren Proteinen in den Fraktionen Niederschlag IV, Niederschlag B, Überstand c und Überstand GG oder in den entsprechenden Fraktionen der Plasmafraktionierung nach Cohn (Fraktion IV-1, Fraktion II+III, Überstand V und Überstand 11-1,2) zu finden sind. Aus ethischen Gründen, aber auch wegen weltweitem Mangel an menschlichem Blutplasma und gewissen Plasmakomponenten, ist eine Verbesserung der bisher nicht sehr hohen Ausbeute an Immunglobulinen anzustreben.

Immunglobuline spielen bei der Abwehr von Infektionen eine zentrale Rolle. Entweder blockieren Virus-spezifische, neutralisierende Antikörper die Adsorption von Viren an die zellulären Rezeptoren und verhindern so eine Infektion, oder dann opsonisieren Bakterien-spezifische Antikörper die Erreger und ermöglichen so deren Eliminierung und Abtötung durch Neutrophile und Makrophagen. Plasmapools aus mehreren tausend Spenden enthalten Immunglobuline sehr vieler verschiedener Spezifitäten, und Immunglobulinpräparate aus solchen Pools enthalten demzufolge auch messbare Titer an Immunglobulinen, die gegen Epitope auf Viren, Bakterien, Toxinen, aber auch gegen Autoantigene gerichtet sind. Sie sind demzufolge gegen viele Infektionen und in verschiedensten weiteren pathologischen Zuständen wirksam. Unter bestimmten Umständen ist es nun aber wünschenswert, ein Immunglobulinpräparat mit hohen Titern an spezifischen Antikörpern, ein sogenanntes Hyperimmunglobulinpräparat, anzuwenden. Bisher wurden solche Präparate mit grossem Aufwand und unter hohen Kosten aus speziellen Plasmapools von Spendern mit erhöhten Titern an spezifischen Antikörpern hergestellt. Dies ist aus verschiedenen Gründen mit Schwierigkeiten verbunden. Falls, wie im Falle eines Anti-*Hepatitis B*-Präparates, anerkannte Impfverfahren vorliegen, müssen die Spender geimpft, ausgewählt und die Spenden separat aufgearbeitet werden. Bei vielen anderen Indikationen kann jedoch eine Immunisierung der Spender aus ethischen Gründen nicht erfolgen. Nur selten ist es hier möglich, durch aufwendige Auswahl der Spender (z.B. nach dem Durchmachen einer spezifischen Krankheit) hochtitrige Spenden zu ermitteln und durch deren Verarbeitung ein Präparat zu erhalten.

In EP-A-0 201 004 wird die Herstellung eines polyvalenten Hyperimmunglobulinpräparates beschrieben, wobei es nach herkömmlichen Methoden der Immunglobulinpräparation aus Plasmen, Seren oder Vollblut von Spendern erhalten wird, die wegen ihres relativ hohen Titers gegen das e.coli J5- Antigen oder gegen das Lipid-A-Antigen ausgewählt werden.

Gemäss diesem Verfahren wird somit nicht irgendein Plasma oder irgendeine Fraktion verwendet, sondern es müssen sorgfältig Spender ausgewählt werden, welche den gewünschten hohen Titer gegen bestimmte Mikroorganismen aufweisen. In diesem Dokument wird der Fachmann in keiner Weise dazu angeleitet, dass es möglich ist, Hyperimmunoglobuline aus sogenannten Abfallfraktionen zu gewinnen.

US-A-4 232 004 beschreibt ein antikörperspezifisches Immunadsorbens, welches nützlich ist für die Herstellung von spezifischen Antikörpern aus Serum oder aus Serumantikörpern, welche eine grosse Anzahl von verschiedenen Antikörpertitern umfasst. Auch hier werden keine Abfallfraktionen erwähnt. Auch durch dieses Dokument wird eine Fachperson nicht angeleitet, aus Abfallfraktionen, welche aus Standardfraktionsverfahren anfallen, Hyperimmunglobulin zu isolieren und hierzu das beschriebene trägergebundene Immunadsorbens zu verwenden. Das Ziel des in US-A-4 232 004 beschriebenen Gegenstandes besteht darin, ein Mittel für die Isolierung von spezifischen Antikörpern für die Behandlung von spezifischen Krankheiten zur Verfügung zu stellen, da die Konzentration von spezifischen Antikörpern normalerweise zu tief ist, damit eine wirksame Behandlung solcher Krankheiten durchgeführt werden kann.

In keinem der beiden Dokumente wird vorgeschlagen oder erkannt, dass aus Restfraktionen von Standardverfahren derartige Präparate herstellbar sind.

Es ist somit Aufgabe der vorliegenden Erfindung ein Verfahren zur Verfügung zu stellen, mit dem wertvolle Immunglobulinpräparate aus den oben genannten, bisher kaum verwerteten Fraktionen und Niederschlägen, die bei industriellen Plasmafraktionierungsverfahren anfallen, zugänglich gemacht und isoliert werden können. Die Präparate, welche Hyperimmunglobulinpräparaten entsprechen, sollen anschliessend zu einem verträglichen, insbesondere i.v. verträglichen, virensicheren, flüssigen oder lyophilisierten Präparat verarbeitet werden können.

Es wurde nun gefunden, dass die Herstellung von Hyperimmunglobulinpräparaten bzw. hochtitrigen Immunglobulinpräparaten nach einem von den bisherigen Verfahren verschiedenen Weg möglich ist. Diese neue Methode verwendet Immunglobuline aus den allgemeinen Plasma-Pools, verbessert durch Verwendung von "Abfall"-Fraktionen die Ausnützung des wertvollen Rohstoffes Blutplasma und erlaubt sogar die Herstellung von Hyperimmunglobulinpräparaten mit weit höheren spezifischen Aktivitäten als sie die bisherigen Präparate aufweisen. Dies bedeutet, dass mit kleinen infundierten Volumen und geringen Mengen an infundierten Proteinen, d.h. entsprechend geringer Belastung des Empfängers, in kurzer Zeit hohe Dosen an spezifischen Immunglobulinen verabreicht werden können. Ermöglicht wird dies im erfindungsgemässen Verfahren durch die Anreicherung der spezifischen Immunglobuline durch Adsorption an immobilisierte Antigene, also durch Verwendung aufgearbeiteter "Abfall"-Fraktionen in affinitätschromatographischen Verfahren.

Gegenstand der vorliegenden Erfindung ist das im Patentanspruch 1 definierte Verfahren.

Im erfindungsgemässen Verfahren wird als erstes eine bei einem Plasmafraktionierverfahren erhaltene Fraktion, insbesondere eine Abfallfraktion derart aufbereitet, dass sie in einer Affinitätschromatographie verwendet werden kann. Je nach Herkunft der Fraktion kann diese Aufarbeitung sehr unterschiedlich aussehen. So kann beispielsweise der Überstand GG aufkonzentriert, gegen einen geeigneten Puffer dialysiert und filtriert werden. Niederschläge oder Filterkuchen (Listen von Beispielen für solche Ausgangsmaterialien sind in den Tabellen 1 und 2 aufgeführt) dagegen müssen zuerst in geeigneter Weise, das heisst beispielsweise durch Variation der lonenstärke, des pH, der Temperatur, durch Zugabe von Detergenzien und Salzen, derart suspendiert werden, dass Immunglobuline gezielt solubilisiert werden. Beispielsweise können sie in einem pH-Bereich von 3.0-9.0, bei einer Leitfähigkeit von 5-20 mS/cm, bei 4°C über Nacht gerührt und anschliessend durch Zentrifugation und/oder Filtration geklärt werden. Sowohl Überstände als auch Suspensionen können auf dieser Stufe einem Vireninaktivierungsverfahren nach der Lösungsmittel/Detergens-Methode von Horowitz (Thrombosis and Haemostasis, 65, 1163, 1991), nach der Methylenblau- (Mohr et al. Infusionsther. Infusionsmed. 20, 19 [1993]) oder einer anderen Methode unterworfen werden. Die Immunglobuline können nach der Suspension auch einer Vorreinigung durch Voradsorption an die Säulenmatrix oder durch Behandlung mit einem geeigneten Filterhilfsmittel oder Adsorbens wie z.B. Aluminiumhydroxid, einer Chromatographie über Protein A oder G zur Anreicherung oder einer oder mehreren Ausfällungen mit den gängigen Methoden der Ammoniumsulfat-, der Polyethylenglycol- oder der Ethanol-Fällung oder Kombinationen davon zur Anreicherung, Konzentrierung oder Abreicherung störender Komponenten unterworfen werden.

**Tabelle 1:**

| Beispiele für mögliche Ausgangsmaterialien, die aus der Fraktionierung nach Kistler und Nitschmann oder aus weiteren Aufarbeitungsschritten stammen, die bei der Herstellung von i.v. verträglichen, stabilen Plasmaprodukten anfallen. | |
|---|---|
| Überstände | Niederschläge und Rückstände |
| Überstand c | Niederschlag B |
| Überstand GG | Niederschlag IV |
| | Rückstand nach DEAE-Behandlung |
| | Rückstand nach Aluminiumhydroxid-Behandlung |
| | Rückstände nach Klärfiltrationen I, II und III |

**Tabelle 2:**

| Beispiele für mögliche Ausgangsmaterialien, die aus der Fraktionierung nach Cohn oder aus weiteren Aufarbeitungsschritten stammen,die bei der Herstellung von i.v. verträglichen, stabilen Plasmaprodukten anfallen. | |
|---|---|
| Überstände | Niederschläge und Rückstände |
| Überstand V | Niederschlag II+III |
| Überstand II-1,2 | Niederschlag IV-1 |
| | Rückstand nach DEAE-Behandlung |
| | Rückstand nach Aluminiumhydroxid-Behandlung |
| | Rückstände nach Klärfiltrationen I, II und III |

In der nachstehenden Liste sind Beispiele von möglichen Liganden für die erfindungsgemässe Affinitätschromatigraphie angegeben:
Antigene Determinanten von
   Haemophilus influenza b
   Staphylococcus aureus
   Staphylococcus epidermidis
   Staphylococcus agalactiae
   Streptococcus pneumoniae
   Streptococcus pyogenes
   und weitere pathogene Bakterienstämme

   Tetanus Toxin
   Staphylococcus aureus toxic shock Toxin
   und weitere pathogene Bakterien- oder andere Toxine

   Hepatitis A Virus
   Hepatitis B Virus
   Hepatitis C Virus
   Varizella Zoster Virus
   Cytomegalo Virus
   Respiratory Syncytial Virus
   Parvovirus B19
   Herpes Simplex Virus 1
   Herpes Simplex Virus 2
   Tollwut Virus
   und weitere pathogene Viren

   CD2, CD3, CD4,
   CD5, CD28, CD40, CD72
   ICAM, LFA-1, LFA-3,
   DNA
   und weitere potentielle humane Autoantigene

Nachdem Affinitätsgele durch Immobilisieren von modifizierten oder unmodifizierten Liganden (Beispiele für solche Liganden sind in obiger Liste aufgeführt.) mittels bekannter Methoden hergestellt wurden, werden sie mit den aufbereiteten Überständen und Suspensionen in konzentrierter oder verdünnter Form beladen, wenn notwendig auch durch wiederholtes Applizieren des Durchflusses. Wenn erwünscht, können verschiedene Affinitätsgele nacheinander mit der gleichen Suspension beladen werden. Die Gele werden anschliessend derart gewaschen, dass unspezifisch bindende Proteine überwiegend oder in ausreichendem Masse entfernt werden. Dies kann beispielsweise durch Erhöhen der Salzkonzentration, durch Zugabe eines Detergens und/oder Verschieben des pH-Wertes in der Waschlösung geschehen. Die gebundenen Proteine werden nun von den Liganden abgelöst, beispielsweise durch Elution bei niedrigem oder hohem pH, durch Zugabe von chaotropen Salzlösungen wie Natriumthiocyanat oder Magnesiumchlorid, denaturierenden Agenzien wie SDS oder Harnstoff, Lösungsmitteln wie Ethylenglycol, durch Verändern der Temperatur oder durch Kombinationen hiervon.

Unter Umständen kann es wünschenswert sein, dass die zu immobilisierenden Liganden durch Mutagenese oder chemische oder physikalische Methoden derart modifiziert werden, dass zwar die spezifischen Immunglobuline immer noch an ihre Epitope binden können, jedoch mit verminderter Affinität, so dass die Elution unter milderen Bedingungen erfolgen kann, als mit den unveränderten Liganden. Eine Modifikation der Liganden kann auch erfolgen, um ihre Immobilisierung und/oder ihre Epitop-Präsentierung zu erleichtern und zu verbessern. Technische Einzelheiten und Grundlagen des Affinitätschromatographieverfahrens im allgemeinen sind beschrieben in: Cuatrecasas, P. and Anfinsen, C.B. [1971] Ann. Rev. Biochem. 40, 259; Kull, F.C. and Cuatrecasas, P. [1981] J. Immunol. 126, 1279; Liebing et al. [1994] Vox Sang. 67, 117.

Die spezifischen, abgelösten Immunglobuline werden, nach Bedarf auch mit einer zusätzlichen Filtration zur Eliminierung von Viren, zu einem Endprodukt verarbeitet, das vorzugsweise i.v. angewendet werden kann, und das pyrogenfrei, virensicher und mit oder ohne Zugabe von Stabilisatoren wie Albumin, Aminosäuren oder Kohlehydraten in flüssiger oder lyophilisierter Form stabil ist. Es können jedoch auch Formulierungen verwendet werden, die eine i.m. oder eine topische Anwendung ermöglichen.

Die vorliegende Erfindung wird anhand der folgenden Beispiele näher erläutert, ohne dass dadurch eine Einschränkung beabsichtigt ist.

### Beispiel 1:

HBsAg-Sepharose wurde hergestellt, indem 5 mg rekombinantes Hepatitis B Virus-Oberflächenantigen (HBsAg, Abbott Diagnostics) durch Kopplung der primären Aminogruppen an 1 ml aktivierte CH-Sepharose nach der Vorschrift des Herstellers (Pharmacia Biotech, Uppsala, Schweden) immobilisiert wurde. "Placebo"-Sepharose wurde hergestellt, indem das gleiche Kopplungs-Verfahren mit einem weiteren Gel-Aliquot, aber ohne Beigabe von HBsAg durchgeführt wurde. Die Lagerung der fertigen Gele geschah in PBS mit 0.02% NaN₃ bei 4°C.

70 g Niederschlag B aus der Plasma-Fraktionierung nach Kistler-Nitschmann (NB Lot 4.030.216) wurden in 210 ml 100 mM Zitronensäure, pH 4.0, 0.25% Triton X-100, 10 mM N-Ethylmaleimid (NEM), 1 mM Phenylmethylsulfonyl Fluorid (PMSF), über Nacht bei 4°C auf einem RotaryMix-Gerät suspendiert. Nach Abtrennung der unlöslichen Komponenten durch Zentrifugation (5000g, 4°C, 10 Min.) und Filtration (Porengrösse: 1.2 µm) wurden bei neutralem pH nach Horowitz et al. (Thrombosis and Haemostasis, 65, 1163, 1991) 1% tri-n-Butylphosphat (Merck, Darmastadt, Deutschland) und 1% Triton X-100 zugegeben und bei 30°C unter Mischen während 4 h inkubiert. Anschliessend wurde bei 37°C über Nacht eine Phasentrennung durchgeführt, der klare Unterstand abgetrennt und durch ein 0.45 µm Filter filtriert und bei 4°C gelagert.

130 ml einer solchen NB-Suspension wurden mit 280 ml PBS (Phosphat-gepufferte Salzlösung: 150 mM NaCI, 10 mM Natriumphosphat, pH 7.1) verdünnt und bei 4°C derart über über die Placebo- und anschliessend über die HBsAg-Sepharose-Säule gepumpt, dass der Säulen-Durchfluss wieder ins Vorratsgefäss gelangte. Die Durchflussgeschwindigkeit betrug 8 ml/h während 144 h. Die NB-Suspension wurde somit gesamthaft drei Mal über beide Säulen gepumpt. Nach 90 h wurde die Beladung unterbrochen und die Säulen wurden einzeln zuerst mit PBS und anschliessend mit 200 mM NaCl, 50 mM Tris-HCI pH 7.4 gewaschen, bis die Waschlösung eine optische Dichte bei 280 nm (OD₂₈₀) aufwies, die kleiner als 0.01 war. Nach Abschluss der Beladung wurde wiederum mit PBS und 200 mM NaCl, 50 mM Tris-HCI pH 7.4 gewaschen. Gebundene Proteine wurden mit 5 ml 200 mM Glycin-HCI pH 2.5 abgelöst, sofort neutralisiert und aufgearbeitet. Die Resultate wurden in Tabelle 3 zusammengestellt.

**Tabelle 3:**

| Anti-HBsAg-Affinitätschromatographie mit NB-Suspension | | |
|---|---|---|
| Gel | HBsAg | Placebo |
| Beladung total: | | |
| Protein | 1775 mg | 1775 mg |
| IgG | 450 mg | 450 mg |
| Anti-HBs IgG | 4560 mIU | 4560 mIU |

| Durchlauf: | | |
|---|---|---|
| Anti-HBs IgG | 1140 mIU | 1140 mIU |

| Eluat: | | |
|---|---|---|
| IgG | 0.13 mg | 0.08 mg |
| Anti-HBs IgG | 5603 mIU | 114 mIU |

### Beispiel 2:

Es wurde ausgehend von der Cohn Fraktion II+III (an Stelle von Blutplasma als Ausgangsmaterial) eine Fraktionierung nach Kistler-Nitschmann durchgeführt. 70 g Niederschlag B (NB Lot 4.044.488) aus dieser Fraktionierung nach Kistler-Nitschmann wurden in 210 ml 100 mM Zitronensäure, pH 4.0, 0.25% Triton X-100, 10 mM NEM, 1 mM PMSF, über Nacht bei 4°C auf einem RotaryMix-Gerät suspendiert. Nach Klärung und teilweiser Delipidierung durch Ultrazentrifugation (100'000g, 3h, 4°C: die klare Phase wurde durch seitliches Anstechen des Röhrchens mit einer Kanüle entnommen) wurde die Suspension filtriert (0.45 µm) und bei 4°C gelagert.

125 ml dieser NB-Suspension wurde mit 375 ml PBS verdünnt, das pH mit 0.1 M NaOH auf 7.1 eingestellt, filtriert, und mit 14.5 ml/h, analog zum Beispiel 1, zuerst über Placebo- und anschliessend über HBsAg-Sepharose-Gele gepumpt, die analog zum Beispiel 3 hergestellt worden waren. Anschliessend wurden die Gele separat mit PBS und 200 mM NaCl, 50 mM Tris-HCI pH 7.4 gewaschen. Die gebundenen Proteine wurden mit 5 ml 200 mM Glycin-HCI pH 2.5 abgelöst. Tabelle 4 gibt eine Zusammenstellung der Daten.

**Tabelle 4:**

| Anti-HBsAg-Affinitätschromatographie mit NB-Suspension (Cohn II+III) | | |
|---|---|---|
| Gel | HBsAg | Placebo |
| Beladung total: | | |
| Protein | 1400 mg | 1400 mg |
| IgG | 619 mg | 619 mg |
| Anti-HBs IgG | 10 IU | 10 IU |

| Durchlauf: | | |
|---|---|---|
| Anti-HBs IgG | 5 IU | 5 IU |

| Eluat: | | |
|---|---|---|
| IgG | 0.18 mg | 0.35 mg |
| Anti-HBs IgG | 3.3 IU | 0.08 IU |

### Beispiel 3:

30 Liter Überstand GG (Lot Nr. X95.31.286.1) wurden in PBS diafiltriert und auf 500 ml aufkonzentriert. Entsprechend dem Beispiel 1 wurde das Konzentrat mit 21 ml/h während 118 h über Placebo- und HBsAg-Säule gepumpt. Waschen der Säulen und Ablösen der gebundenen Proteine geschah ebenfalls analog zum Beispiel 1, jedoch wurde ein zusätzlicher Waschschritt mit 500 mM NaCI, 50 mM Tris-HCI pH 7.4 durchgeführt. Die Resultate sind in Tabelle 5 dargestellt.

**Tabelle 5:**

| Anti-HBsAg-Affinitätschromatographie mit Überstand GG-Konzentrat NG: Nachweisgrenze | | |
|---|---|---|
| Gel | HBsAg | Placebo |
| Beladung total: | | |
| Protein | 8800 mg | 8800 mg |
| IgG | 320 mg | 320 mg |
| Anti-HBs IgG | 5000 mlU | 5000 mlU |

| Durchlauf: | | |
|---|---|---|
| Anti-HBs IgG | <NG | <NG |

| Eluat: | | |
|---|---|---|
| IgG | 0.05 mg | 0.14 mg |
| Anti-HBs IgG | 3035 mlU | 7 mIU |

### Beispiel 4:

17.5 g DEAE-Filterkuchen (Lot 4.422.006.0) wurden in 52.5 ml Suspensions-Puffer nach Beispiel 1 suspendiert und aufgearbeitet. 40 ml Suspension wurden mit 160 ml PBS verdünnt, das pH auf 7.1 eingestellt und anschliessend filtriert. Entsprechend dem Beispiel 3 wurde die Suspension mit 21 ml/h während 97 h über Placebo- und HBsAg-Säule gepumpt. Waschen der Säulen und Ablösen der gebundenen Proteine geschah ebenfalls analog zum Beispiel 1. Die Resultate werden in Tabelle 6 dargestellt.

**Tabelle 6:**

| Anti-HBsAg-Affinitätschromatographie mit DEAE-Filterkuchen-Suspension NG: Nachweisgrenze | | |
|---|---|---|
| Gel | HBsAg | Placebo |
| Beladung total: | | |
| Protein | 548 mg | 548 mg |
| IgG | 280 mg | 280 mg |
| Anti-HBs IgG | 400 mIU | 400 mlU |

| Durchlauf: | | |
|---|---|---|
| Anti-HBs IgG | <NG | <NG |

| Eluat: | | |
|---|---|---|
| IgG | 0.07 mg | 0.11 mg |
| Anti-HBs IgG | 331 mlU | 14 mlU |

### Beispiel 5:

Tetanus Toxoid-C-Sepharose wurde hergestellt, indem 11.5 mg gereinigtes Tetanus Toxoid (TT) durch Kopplung der Carboxygruppen an 1 ml EAH-Sepharose (Pharmacia Biotech, Uppsala, Schweden) mit Hilfe von 0.1 M N-Ethyl-N'-(3-dimethylaminopropyl)-carbodiimid-HCI nach der Vorschrift des Herstellers immobilisiert wurde. Die Lagerung der fertigen Gele geschah in PBS mit 0.02% NaN₃ bei 4°C.

Ein Konzentrat aus Überstand GG wurde analog zu Beispiel 3 aufbereitet und zur Affinitätschromatographie mit TT-Sepharose analog zu Beispiel 2 verwendet. Die Beladung erfolgte mit 23.5 ml/h während 159 h bei 4°C. Die Resultate werden in Tabelle 7 dargestellt.

**Tabelle 7:**

| Anti-Tetanus Toxoid-Affinitätschromatographie mit Überstand GG-Konzentrat | |
|---|---|
| Gel | Tetanus Toxoid C |
| Beladung total: | |
| Protein | 15000 mg |
| IgG | 320 mg |
| Anti-TT IgG | 36 µg |

| Durchlauf: | |
|---|---|
| Anti-TT IgG | 16 µg |

| Eluat: | |
|---|---|
| IgG | 0.16 mg |
| Anti-TT IgG | 76 µg |

### Beispiel 6:

Tetanus Toxoid-N-Sepharose wurde hergestellt, indem 11.5 mg gereinigtes Tetanus Toxoid (TT) durch Kopplung der primären Aminogruppen an 1 ml aktivierte CH-Sepharose nach der Vorschrift des Herstellers (Pharmacia Biotech, Uppsala, Schweden) immobilisiert wurde. "Placebo"-Sepharose wurde hergestellt, indem das gleiche Kopplungs-Verfahren mit einem weiteren Gel-Aliquot, aber ohne Beigabe von TT durchgeführt wurde. Die Lagerung der fertigen Gele geschah in PBS mit 0.02% NaN₃ bei 4°C.

Niederschlag B wurde gemäss Beispiel 2 suspendiert. Nach Filtration (1.2 µm) wurden 115 ml dieser Suspension mit 200 ml PBS verdünnt, das pH auf 7.1 eingestellt und mit 3.5 ml/h während 165 h derart zuerst über die Placebo-Sepharose gepumpt, dass deren Durchfluss unmittelbar anschliessend über die TT-N-Sepharose-Säule und danach wieder ins Vorratsgefäss gelangte. Die Säulen wurden separat mit PBS und darauf mit 0.5 M NaCI, 50 mM Tris-HCl pH 7.1 gewaschen, bis die OD₂₈₀ des Durchflusses kleiner als 0.01 war. Die Resultate werden in Tabelle 8 dargestellt.

**Tabelle 8:**

| Anti-Tetanus Toxoid-Affinitätschromatographie mit NB-Suspension | | |
|---|---|---|
| Gel | Tetanus Toxoid N | Placebo |
| Beladung total: | | |
| Protein | 1334 mg | 1334 mg |
| IgG | 333 mg | 333 mg |
| Anti-TT IgG | 0.509 mg | 0.509 mg |

| Durchlauf: | | |
|---|---|---|
| Anti-TT IgG | 0.213 mg | 0.213 mg |

| Eluat: | | |
|---|---|---|
| IgG | 0.35 mg | 0.004 mg |
| Anti-TT IgG | 0.091 mg | 0.003 mg |

### Beispiel 7:

Niederschlag B wurde gemäss Beispiel 2 suspendiert. Nach Filtration (1.2 µm) wurden 115 ml dieser Suspension mit 200 ml PBS verdünnt, das pH auf 7.1 eingestellt und mit 5 ml/h während 165 h derart zuerst über die HBsAg-Sepharose gepumpt, dass deren Durchfluss unmittelbar anschliessend über die TT-Sepharose-Säule und danach wieder ins Vorratsgefäss gelangte. Die Säulen wurden separat mit PBS und darauf mit 0.5 M NaCI, 50 mM Tris-HCI pH 7.1 gewaschen, bis die OD₂₈₀ des Durchflusses kleiner als 0.01 war. Die Resultate werden in Tabelle 9 dargestellt.

**Tabelle 9:**

| Anti-HBsAg- und Anti-Tetanus Toxoid-Affinitätschromatographie mit NB-Suspension | | |
|---|---|---|
| Gel | HBsAg | Tetanus Toxoid |
| Beladung total: | | |
| Protein | 1334 mg | 1334 mg |
| IgG | 333 mg | 333 mg |
| Anti-HBsAg | 945 mlU | 945 mIU |
| Anti-TT IgG | 0.509 mg | 0.509 mg |

| Durchlauf: | | |
|---|---|---|
| Anti-HBsAg | 536 mIU | 536 mIU |
| Anti-TT IgG | 0.265 mg | 0.265 mg |

| Eluat: | | |
|---|---|---|
| IgG | 0.13 mg | 0.25 mg |
| Anti-HBsAg | 1152 mIU | |
| Anti-TT IgG | | 0.18 mg |

### Beispiel 8:

15 kg Niederschlag B aus der Fraktionierung nach Kistler-Nitschmann (Lot Nr. 5.043.303) wurden in 45 Liter 0.1 M Zitronensäure, pH 4.0, 0.25% Triton X-100, 10 mM NEM, 1 mM PMSF bei 4°C über Nacht mit einem Vibromischer suspendiert. Nach Abtrennung der unlöslichen Komponenten durch Zugabe von Filterhilfsmittel und Filtration (Porengrösse: 1.2 µm) wurden bei neutralem pH zur Delipidierung und Vireninaktivierung nach Horowitz 1% tri-n-Butylphosphat und 1% Triton X-100 zugegeben und bei 30°C unter Mischen während 4 h inkubiert. Anschliessend wurde bei 37°C über Nacht eine Phasentrennung durchgeführt, der klare Unterstand abgepumpt, durch ein 0.45 µm Filter filtriert und bei 4°C gelagert.

Das pH von 40 Litern dieser NB-Suspension wurde mit NaOH auf einen Wert von 7.1 gebracht und bei 4°C derart über eine HBsAg- und eine Tetanus Toxoid-Affiprep-Säule gepumpt, dass der Säulen-Durchfluss wieder ins Vorratsgefäss gelangte. Die Affiprep-Säulen (50x13 mm) waren durch Kopplung von 250 mg rekombinantem HBsAg bzw. Tetanus Toxoid an 25 ml Affiprep-Gel (BioRad Lab. Inc. Hercules CA 94547) hergestellt worden. Die Durchflussgeschwindigkeit betrug 6 Liter/h während 62 h. Die NB-Suspension wurde somit gesamthaft drei Mal über die Säulen gepumpt. Nach Abschluss der Beladung wurden die Säulen separat zuerst mit PBS und anschliessend mit 500 mM NaCl, 50 mM Tris-HCI pH 7.4 gewaschen, bis die Waschlösungen eine optische Dichte bei 280 nm (OD₂₈₀) aufwies, die kleiner als 0.01 war. Gebundene Proteine wurden mit 200 mM Glycin-HCI pH 2.5 abgelöst und das pH der Fraktionen sofort auf 5.2 eingestellt. Die Resultate wurden in Tabelle 10 zusammengestellt. Die Aufarbeitung der Immunglobuline zu stabilen Präparaten geschah durch Poolen der IgG enthaltenden Fraktionen, Diafiltration und Konzentration mit 20 mM NaCI zu Lösungen von 100 IU/ml, bzw. 2.5 mg anti-TT-IgG/ml. 10% Saccharose wurde zugefügt und die Lösungen in Einheiten zu 200 IU, bzw. 5 mg anti-TT-IgG lyophilisiert.

### Beispiel 9:

50 g Niederschlag IV aus der Plasma-Fraktionierung nach Kistler-Nitschmann wurden in 500 ml Wasser suspendiert. Der pH-Wert wurde mit Zitronensäure auf 5.0 und die Leitfähigkeit mit NaCl auf 13 mS eingestellt. Nach Rühren über Nacht bei 4°C wurde durch Zentrifugieren während 30 min bei 30'000 g und 4°C eine Klärung und teilweise Delipidierung erreicht. Eine Bestimmung des Gehaltes der Suspension an gelösten Proteinen, IgM, IgA, IgG, Transferrin und Caeruloplasmin wurde durchgeführt und in Tabelle 11 zusammengestellt.

**Tabelle 10:**

| Anti-HBsAg- und Anti-Tetanus Toxoid-Affinitätschromatographie mit NB-Suspension | | |
|---|---|---|
| Gel | HBsAg | Tetanus Toxoid |
| Beladung total: | | |
| Protein | 448 g | 448 g |
| IgG | 204 g | 204 g |
| Anti-HBs IgG | 1120 IU | 1120 IU |
| Anti-TT IgG | 317 mg | 317 mg |

| Durchlauf: | | |
|---|---|---|
| Anti-HBs IgG | 221 IU | 221 IU |
| Anti-TT IgG | 172 mg | 172 mg |

| Eluat: | | |
|---|---|---|
| IgG | 39.8 mg | 171 mg |
| Anti-HBs IgG | 1368 IU | |
| Anti-TT IgG | | 89 mg |

**Tabelle 11:**

| Suspension aus Niederschlag IV (Alle Angaben in mg/ml) | | | | | |
|---|---|---|---|---|---|
| Total Protein | IgM | IgA | IgG | Transferrin | Caeruloplasmin |
| 5.5 | 0.063 | 0.628 | 0.488 | 2.582 | 0.09 |

### Beispiel 10:

50g Niederschlag B aus der Plasma-Fraktionierung nach Kistler-Nitschmann (Lot 4.030.204.0) wurde in 100 mM Zitronensäure bei verschiedenen pH Werten bei 4°C über Nacht gerührt, durch Ultrazentrifugation bei 100'000 g und 4°C während 3 h geklärt und teilweise delipidiert. Die klare mittlere Phase wurde entnommen und deren Gehalt an gelösten Proteinen, IgM, IgA, IgG, Transferrin und Caeruloplasmin bestimmt (Tabelle 12).

**Tabelle 12:**

| Suspension aus Niederschlag B (Alle Angaben in mg/ml); <NG: unter Nachweisgrenze | | | | | | |
|---|---|---|---|---|---|---|
| | Total Protein | IgM | IgA | IgG | Transferrin | Caeruloplasmin |
| pH 4.0 | 4.9 | 1.2 | 1.3 | 1.9 | <NG | 0.06 |
| pH 5.0 | 6.1 | 1.2 | 1.0 | 2.1 | <NG | <NG |

Der Titer von IgG gegen bestimmte virale (Tabelle 13) und bakterielle (Tabelle 14) Antigene wurde bestimmt und mit demjenigen in Ausgangsplasma und bestehenden Immunglobulinpräparaten verglichen.

**Tabelle 13:**

| Antivirale IgG in Plasmapools, SAGL und NB-Suspensionen | | | | | | |
|---|---|---|---|---|---|---|
| | Plasma 102 | Plasma 103 | SAGL | NB pH 4.0 | NB pH 5.0 | |
| Anti-HBsAg | 0.02 | 0.05 | 0.01 | 0.05 | 0.05 | IU/mg IgG |
| Anti-CMV | 0.28 | 0.16 | 0.3 | 0.52 | 0.34 | PEIE/mg IgG |
| Anti-VZV | 0.08 | 0.09 | n.b. | 0.21 | 0.1 | IU/mg IgG |
| Anti-Masern | 0.9 | 0.2 | 0.02 | 2.1 | 0.81 | IU/mg IgG |
| Anti-HSV1 | 1037 | n.b. | n.b. | 2317 | 749 | AU/mg IgG |

Plasma 102/103: Plasmapools; SAGL: Sandoglobulin; NB pH 4.0/5.0: Suspension aus Niederschlag B bei pH 4.0 resp. 5.0; IU: Internationale Einheiten; PEIE: Paul Ehrlich Institut-Einheiten; AU: Arbiträre Einheiten; n.b.: nicht be stimmt.

**Tabelle 14:**

| Antibakterielle IgG in Plasmapools, SAGL und NB-Suspensionen | | | | | | |
|---|---|---|---|---|---|---|
| | Plasma 102 | Plasma 103 | SAGL | NB 4A | NB 14 pH 4.0 | |
| anti-HiBOAg | 162 | 286 | 436 | 100 | 283 | µg/g IgG |
| anti-TT | 1855 | 4159 | 2740 | 2928 | 2923 | µg/g IgG |
| anti-SEB | 412 | 689 | 783 | 918 | 670 | AU/g IgG |

Plasma 102/103: Plasmapools; SAGL: Sandoglobulin ®; NB 4A: Suspension aus Niederschlag B bei pH 4.0; NB 14 pH 4.0: Suspension aus Niederschlag B bei pH 4.0 mit Virusinaktivierung; HiBOAg: Haemophilius influenza B-Oligosaccharid-Antigen: TT: Tetanus Toxoid SEB: Staph<lococcus Enterotoxin B; AU: Arbiträre Einheiten.

## Patentansprüche

1. Verfahren zur Herstellung eines hochtitrigen Immunglobulin-Präparates, **dadurch gekennzeichnet, dass** ausgehend von Abfallfraktionen, die bei den industriellen Plasmafraktionierungsverfahren anfallen, folgende Schritte nacheinander durchgeführt werden:
a) Herstellung einer Proteinlösung aus den Proteinkomponenten, die in der Abfallfraktion enthalten sind, und
b) Unterwerfung der resultierenden Proteinlösung mindestens einmal einer Affinitätschromatographie mit immobilisierten Liganden von mindestens einem Ligandentyp, wobei die spezifischen Plasmaproteine an die Liganden gebunden werden, und Ablösung der gebundenen Plasmaproteine, welche als aktive Komponenten in ein hochtitriges Immunglobulin-Präparat übergeführt werden.

2. Verfahren nach Anspruch 1, **dadurch gekennzeichnet, dass** die Abfallfraktion ein Niederschlag oder ein Filterkuchen ist und die Proteinkomponenten in Lösung gebracht werden, indem der Niederschlag oder der Filterkuchen mit einer wässerigen Pufferlösung mit einer lonenstärke von <5M und einem pH-Wert von 3,0 bis 9,0 unter Bildung einer Lösung oder Suspension behandelt wird.

3. Verfahren nach Anspruch 2, **dadurch gekennzeichnet, dass** die Pufferlösung ein Phosphatpuffer, ein Tris-HCl-Puffer oder ein Citratpuffer ist.

4. Verfahren nach Anspruch 2 oder 3, **dadurch gekennzeichnet, dass** die Pufferlösung ein Detergens, einen Proteasehemmer und/oder ein Salz enthält.

5. Verfahren nach einem der Ansprüche 2 bis 4, **dadurch gekennzeichnet, dass** die Lösung oder Suspension filtriert oder mit einem Adsorbens, wie Aluminiumhydroxid oder einem Adsorbens, das DEAE-Gruppen enthält, vorbehandelt wird.

6. Verfahren nach einem der Ansprüche 2 bis 5, **dadurch gekennzeichnet, dass** die Lösung oder Suspension unter Bildung einer Ausfällung mit Ammoniumsulfat, Polyethylenglykol oder Ethanol versetzt wird, wobei der Überstand oder die Ausfällung weiterverarbeitet wird.

7. Verfahren nach Anspruch 1 oder 2, **dadurch gekennzeichnet, dass** die Restfraktion gemäss Stufe a) ein Überstand ist und die Herstellung der Proteinlösung durch Filtration und Konzentration, beispielsweise durch Diafiltration, erfolgt.

8. Verfahren nach Anspruch 7, **dadurch gekennzeichnet, dass** der Überstand filtriert oder mit einem Adsorbens, wie Aluminiumhydroxid oder einem Adsorbens, das DEAE-Gruppen enthält, vorbehandelt wird.

9. Verfahren nach einem der Ansprüche 1 bis 8, **dadurch gekennzeichnet, dass** die immobilisierten Liganden natürliche oder rekombinante, virale bakterielle oder zelluläre Antigene sind.

10. Verfahren nach Anspruch 9, **dadurch gekennzeichnet, dass** die Liganden ausgewählt sind aus der Gruppe der antigenen Determinanten von Haemophilus influenza b, Staphylococcus aureus, Staphylococcus epidermidis, Staphylococcus agalactiae, Streptococcus pneumoniae, Streptococcus pyogenes, Tetanus Toxin, Staphylococcus aureus toxic shock Toxin, Hepatitis A Virus, Hepatitis B Virus, Hepatitis C Virus, Varizella Zoster Virus, Cytomegalo Virus, Respiratory Syncytial Virus, Parvovirus B19, Herpes Simplex Virus 1 und 2, Tollwut Virus, sowie den potentiellen humanen Autoantigenen CD2, CD3, CD4, CD5, CD28, CD40, CD72 ICAM, LFA-1, LFA-3, DNA und Phospholipide.

11. Verfahren nach Anspruch 9 oder 10, **dadurch gekennzeichnet, dass** die Liganden durch Mutagenese oder chemische oder physikalische Methoden modifiziert wurden.

12. Verfahren nach einem der Ansprüche 1 bis 11, **dadurch gekennzeichnet, dass** das erhaltene hochtitrige Immunglobulinpräparat nur aus Immunglobulinen der Klasse G oder A oder M oder aus beliebigen Kombinationen davon besteht.

13. Verfahren nach einem der Ansprüche 1 bis 12, **dadurch gekennzeichnet, dass** das erhaltene hochtitrige Immunglobulinpräparat einer Vireninaktivierung unterworfen wird und erforderlichenfalls stabilisiert wird, wobei ein Stabilisator, wie Albumin, Aminosäuren oder Kohlenhydrate, zugesetzt werden und/oder das Produkt lyophilisiert wird.

14. Verfahren nach einem der Ansprüche 1 bis 13, **dadurch gekennzeichnet, dass** das erhaltene hochtitrige Immunglobulinpräparat in ein pharmazeutisch annehmbares Produkt übergeführt wird, beispielsweise in ein i.v., i.m. oder topisch verabreichbares Präparat.

## Claims

1. Method of producing a high-titre immunoglobulin preparation, **characterised in that**, beginning with waste fractions which arise during industrial plasma fractionation processes, the following steps are carried out in succession:
(a) preparing a protein solution from the protein components which are contained in the waste fraction, and
(b) subjecting the resultant protein solution at least once to affinity chromatography with immobilised ligands of at least one ligand type, the specific plasma proteins being bound to the ligands, and separation of the bound plasma proteins, which are converted as active components into a high-titre immunoglobulin preparation.

2. Method according to claim 1, **characterised in that** the waste fraction is a precipitate or a filter cake, and the protein components are put into solution by treating the precipitate or the filter cake with an aqueous buffer solution having an ionic strength of < 5M and a pH of from 3.0 to 9.0, a solution or suspension being formed.

3. Method according to claim 2, **characterised in that** the buffer solution is a phosphate buffer, a tris-HCI buffer or a citrate buffer.

4. Method according to claim 2 or 3, **characterised in that** the buffer solution is a detergent, a protease inhibitor and/or a salt.

5. Method according to one of the claims 2 to 4, **characterised in that** the solution or suspension is filtered or is pre-treated with an adsorbent such as aluminium hydroxide or an adsorbent containing DEAE groups.

6. Method according to one of the claims 2 to 5, **characterised in that** the solution or suspension is reacted with ammonium sulfate, polyethylene glycol, or ethanol, a precipitate being formed, and the supernatant or the precipitate being further processed.

7. Method according to claim 1 or 2, **characterised in that** the residual fraction according to step (a) is a supematant, and preparation of the protein solution takes place by filtration and concentration, e.g. by diafiltration.

8. Method according to claim 7, **characterised in that** the supernatant is filtered or is pre-treated with an adsorbent such as aluminium hydroxide or an adsorbent containing DEAE groups.

9. Method according to one of the claims 1 to 8, **characterised in that** the immobilised ligands are natural or recombinant, viral, bacterial, or cellular antigens.

10. Method according to claim 9, **characterised in that** the ligands are selected from the group of antigen determinants of haemophilus influenza b, staphylococcus aureus, staphylococcus epidermidis, staphylococcus agalactiae, streptococcus pneumoniae, streptococcus pyogenes, tetanus toxin, staphylococcus aureus toxic shock toxin, hepatitis A virus, hepatitis B virus, hepatitis C virus, varizella zoster virus, cytomegalo virus, respiratory syncytial virus, parvovirus B19, herpes simplex virus 1 and 2, rabies virus, and the potential human autoantigens CD2, CD3, CD4, CD5, CD28, CD40, CD72 ICAM, LFA-1, LFA-3, DNA and phospholipids.

11. Method according to claim 9 or 10, **characterised in that** the ligands have been modified by mutagenesis or chemical or physical methods.

12. Method according to one of the claims 1 to 11, **characterised in that** the high-titre immunoglobulin preparation obtained consists only of immunoglobulins of the classes G or A or M or any combination thereof.

13. Method according to one of the claims 1 to 12, **characterised in that** the high-titre immunoglobulin preparation obtained is subjected to virus inactivation and, if necessary, stabilised, a stabiliser such as albumin, amino acids, or carbohydrates being added and/or the product being lyophilised.

14. Method according to one of the claims 1 to 13, **characterised in that** the high-titre immunoglobulin preparation obtained is converted into a pharmaceutically acceptable product, e.g. into an intravenously, intramuscularly, or topically administrable preparation.

## Revendications

1. Procédé de préparation d'une préparation d'immunoglobuline à titre élevé, **caractérisé en ce que**, en partant de fractions de sous-produits obtenues dans des procédés industriels de fractionnement du plasma, on procède successivement aux étapes suivantes :
a) préparation d'une solution de protéines, constituée des composants protéiques contenus dans la fraction de sous-produits, et
b) soumission, au moins une fois, de la solution obtenue de protéines à une chromatographie d'affinité avec des ligands immobilisés appartenant à au moins un type de ligand, où les protéines plasmatiques spécifiques sont liées aux ligands, et élimination, par dissolution, des protéines plasmatiques liées, qui en tant que composants actifs sont converties en une préparation d'immunoglobulines à titre élevé.

2. Procédé selon la revendication 1, **caractérisé en ce que** la fraction de sous-produits est un précipité ou un gâteau de filtration, et les composants protéiques sont mis en solution par traitement du précipité ou du gâteau de filtration avec une solution tampon aqueuse ayant une force ionique < 5 M et un pH de 3,0 à 9,0, avec formation d'une solution ou d'une suspension.

3. Procédé selon la revendication 2, **caractérisé en ce que** la solution tampon est un tampon phosphate, un tampon Tris-HCI ou un tampon citrate.

4. Procédé selon la revendication 2 ou 3, **caractérisé en ce que** la solution tampon contient un détergent, un inhibiteur de protéase et/ou un sel.

5. Procédé selon l'une des revendications 2 à 4, **caractérisé en ce que** la solution ou la suspension est filtrée ou est soumise à un traitement préalable avec un adsorbant tel que l'hydroxyde d'aluminium, ou avec un adsorbant qui contient des groupes DEAE.

6. Procédé selon l'une des revendications 2 à 5, **caractérisé en ce que** la solution ou la suspension est, avec formation d'un précipité, additionnée de sulfate d'ammonium, de polyéthylèneglycol ou d'éthanol, le surnageant ou le précipité subissant un traitement plus poussé.

7. Procédé selon la revendication 1 ou 2, **caractérisé en ce que** la fraction résiduelle de l'étape a) est un surnageant, et que la préparation de la solution de protéines s'effectue par filtration et concentration, par exemple par filtration sur membrane.

8. Procédé selon la revendication 7, **caractérisé en ce que** le surnageant est filtré ou est soumis à un traitement préalable avec un adsorbant tel que l'hydroxyde d'aluminium, ou avec un adsorbant qui contient des groupes DEAE.

9. Procédé selon l'une des revendications 1 à 8, **caractérisé en ce que** les ligands immobilisés sont des antigènes viraux, bactériens ou cellulaires, naturelles ou recombinants.

10. Procédé selon la revendication 9, **caractérisé en ce que** les ligands sont choisis dans l'ensemble comprenant les déterminants antigéniques de Haemophilus influenza b, Staphylococcus aureus, Staphylococcus epidermidis, Staphylococcus agalactiae, Streptococcus pneumoniae, Streptococcus pyogenes, la toxine tétanique, le toxine du choc toxique par Staphylococcus aureus, le virus de l'hépatite A, le virus de l'hépatite B, le virus de l'hépatite C, l'herpesvirus, le virus cytomégalique, le virus respiratoire syncytial, le parvovirus B19, le virus herpes simplex 1 et 2, le virus de la rage, ainsi que les autoantigènes humains potentiels CD2, CD3, CD4, CD5, CD28, CD40, CD72, ICAM, LFA-1, LFA-3, ADN et phospholipides.

11. Procédé selon la revendication 9 ou 10, **caractérisé en ce que** les ligands ont été modifiés par mutagenèse ou par des techniques chimiques ou physiques.

12. Procédé selon l'une des revendications 1 à 11, **caractérisé en ce que** la préparation d'immunoglobulines à titre élevé telle qu'obtenue n'est constitué que d'immunoglobulines de la classe G, ou A, ou M, ou de combinaisons quelconques de ces immunoglobulines.

13. Procédé selon l'une des revendications 1 à 12, **caractérisé en ce que** la préparation d'immunoglobulines à titre élevé telle qu'obtenue est soumise à une inactivation du virus et si nécessaire est stabilisée, ce pour quoi on ajoute un stabilisant tel que l'albumine, des acides aminés ou des hydrates de carbone, et/ou on lyophilise le produit.

14. Procédé selon l'une des revendications 1 à 13, **caractérisé en ce que** la préparation d'immunoglobulines à titre élevé obtenue est convertie en un produit acceptable d'un point de vue pharmaceutique, par exemple en une préparation pouvant être administrée i.v., i.m. ou par voie topique.
